(19) Europäisches Patentamt / European Patent Office / Office européen des brevets

(11) EP 3 265 243 B1

# (12) EUROPEAN PATENT SPECIFICATION

(45) Date of publication and mention of the grant of the patent:
**06.04.2022 Bulletin 2022/14**

(21) Application number: **16706583.8**

(22) Date of filing: **25.02.2016**

(51) International Patent Classification (IPC):
***B06B 1/02*** *(2006.01)*

(52) Cooperative Patent Classification (CPC):
**B06B 1/0292**

(86) International application number:
**PCT/EP2016/053899**

(87) International publication number:
**WO 2016/139103 (09.09.2016 Gazette 2016/36)**

## (54) A CMUT ARRAY COMPRISING AN ACOUSTIC WINDOW LAYER

CMUT-ARRAY MIT EINER SCHALLFENSTERSCHICHT

MATRICE DE TRANSDUCTEUR CAPACITIF ULTRASONORE MICRO-USINÉ COMPRENANT UNE COUCHE FENÊTRE ACOUSTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.03.2015 EP 15157280**

(43) Date of publication of application:
**10.01.2018 Bulletin 2018/02**

(73) Proprietor: **Koninklijke Philips N.V.**
**5656 AG Eindhoven (NL)**

(72) Inventors:
- **BECKERS, Lucas Johannes Anna Maria**
  **5656 AE Eindhoven (NL)**
- **HAKKENS, Franciscus Johannes Gerardus**
  **5656 AE Eindhoven (NL)**
- **DIRKSEN, Peter**
  **5656 AE Eindhoven (NL)**

(56) References cited:
**EP-A2- 0 239 999**
**WO-A2-2009/016606**
**US-A1- 2014 265 728**
**US-B2- 8 343 289**

- **MEIER H ET AL: "Some aspects of a new class of sulfur containing phenolic antioxidants", POLYMER DEGRADATION AND STABILITY, BARKING, GB, vol. 49, no. 1, 1 January 1995 (1995-01-01), pages 1-9, XP027286607, ISSN: 0141-3910 [retrieved on 1995-01-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 265 243 B1

## Description

### FIELD OF THE INVENTION

The invention relates to an ultrasound array for acoustic wave transmission comprising at least one capacitive micro-machined ultrasound transducer (CMUT) cell, wherein the CMUT cell comprises a substrate; a first electrode; a cell membrane opposing the substrate and having a second electrode, which opposes the first electrode with a cavity there between, wherein the membrane is arranged to vibrate upon the cell activation; and an acoustic window layer, overlaying the cell membrane, and having an inner surface opposing the cell membrane and an outer surface.

The invention further relates to a manufacturing method of such an array; and an ultrasonic imaging system comprising such an ultrasound array.

### BACKGROUND OF THE INVENTION

Central to any ultrasound (imaging) array is the ultrasound transducer which converts electrical energy in acoustic energy and back. Recent progress in semiconductor technology resulted in the development of capacitive micro-machined ultrasound transducers (CMUT). These transducers are considered to be potential candidates to replace the conventional piezoelectric based ultrasound transducers (PZT). A CMUT transducer cell comprises a cavity with a movable mechanical part also called a membrane and a pair of electrodes separated by the cavity. When receiving ultrasound waves, ultrasound waves cause the membrane to move or vibrate and change the capacitance between the electrodes which can be detected. Thereby the ultrasound waves are transformed into a corresponding electrical signal. Conversely, an electrical signal applied to the electrodes causes the membrane to move or vibrate, thereby transmitting ultrasound waves.

Advantages of CMUTs are that they can be made using semiconductor fabrication processes and, therefore, may be easier integrated with application-specific integrated circuitry (ASIC); CMUT transducers offer low cost, extended frequency range and finer acoustic pitch over traditional PZTs.

Inherently from the PZT based technology most of the commonly used ultrasound arrays with CMUTs have acoustic windows or lens materials selected from the materials used for the PZT-based transducers, such as silicon rubbers, for example RTV.

However, the CMUT possesses different to the PZT mechanism of an electro-acoustical transformation, wherein an interactions between the CMUT membranes and the acoustic materials used for acoustic window or lens may reduce the acoustic performance of the transducer.

US 2013/0301394 A1 suggests using a coupling medium between the CMUT and the acoustic window. The presented examples of the medium materials contain either solid-based materials, such as plastics, rubber, room temperature vulcanizing silicon (RTV), dry film photoresists; or liquid-based materials, such as oils, gels. A drawback of this coupling medium that the suggested list of the coupling medium materials contains different materials having a broad range of acoustic properties often showing a strong attenuation for the propagating acoustic wave. A selection of the specific medium material would further require its adjustment to an acoustic window layer applied to the ultrasound array.

US 2014/265728 A1 describes a transducer stack of CMUTs with a matching layer, which can be made from a compliant material (e.g. an elastomer and/or a liquid) and configured for use with CMUTs. A first layer the matching layer is suggested to overlay the membrane surface of the CMUT and is made from a compliant material such as a PDMS type silicone, an elastomer, a gel or a polymerized material.

There is a need in providing an improved acoustic window layer suitable for the CMUT-based ultrasound array for acoustic wave transmission, wherein the CMUT cells are adapted to operate in both: conventional and collapsed operation modes.

### SUMMARY OF THE INVENTION

It is an object of the present invention to provide an ultrasound array according to claim 1.

It was discovered that traditional filled silicon rubbers obtained by vulcanization (also referred here as room temperature curing rubber or RTV) acoustic lens materials, which are easily cast in place and formed by molding into a desired shape, brought in contact with the CMUT array introduce an additional acoustic losses in the CMUT array in addition to normal frequency dependent attenuation. This loss is manifested in increased attenuation on the order of 2dB and a downshift in center frequency of up to 4 MHz. It was discovered that the polymeric material containing only hydrogen and carbon atoms may exhibit an acoustic loss per millimeter for acoustic energy passing therethrough of less than 1.5 dB for a wide range of the acoustic wave frequencies applicable in medical ultrasound, such as in between 2 and 25 MHz. A distinguishing feature of the present invention is that the first layer additionally comprises molecules of antioxidant, which prevents the polymeric layer to further cross link (oxidize) with time. Thus, the antioxidant keeps

properties of the polymeric material, such as hardness value, constant in time. The polymeric material, which contains carbon and hydrogen atoms only and has a density equal or below 0.95 g/cm$^3$ and an acoustic impedance equal or above 1.4 MRayl, placed in a direct contact with the cell membrane provides an improved acoustical coupling of the acoustic window layer to the membrane of the CMUT cell. Thus, no additional coupling medium between the acoustic window and the CMUT array is required.

In an embodiment of the present invention the polymeric material comprises a thermoset elastomer.

The thermoset rubber, also known as thermoset elastomer, containing only hydrogen and carbon atoms have a relatively low densityand in an uncured state have a hardness value below 50 Shore A (typical to the elastomers). These properties combined with the low acoustic wave attenuation may provide a beneficial effect on the improved acoustic coupling of the CMUT vibrating membranes with the acoustic window layer. In addition, the elastomer's acoustic impedance values are closer to the tissue's impedance, which may lead to a better acoustic impedance matching between the acoustic window layer and the tissue.

In yet another embodiment of the present invention the thermoset elastomer is polybutadiene.

Polybutadiene belongs to the thermoset rubbers (elastomers) containing no other atom types than hydrogen and carbon (hydrocarbons). This material shows one of the lowest attenuation effects on the propagating acoustic energy. Additionally polybutadiene material provides a large band width of about 140% at 3 dB-point for the propagating acoustic signal. The antioxidant molecules keep the hardness of the first layer comprising polybutadiene around 5 Shore, well below 50 Shore. The acoustical coupling of the acoustic window layer to the membrane of the CMUT provides an optimal preservation of mechanical properties of the vibrating (moving) part and results in the optimal acoustic energy propagation.

In a further embodiment of the present invention the thermoset elastomer is a copolymer, such as butyl rubber.

Copolymers comprising two monomers provide a further possibility for the acoustic layer's attenuation and hardness adjustment via inheriting some of the beneficial acoustic properties from one of the monomers, such as isoprene. In particular, butyl rubber is an isobutylene-isoprene copolymer and shows hardness as low as 40 ShoreA.

In another embodiment the molecules of antioxidant are phenolic stabilizers, wherein each molecule of the phenolic stabilizer has a hydrocarbon chain coupled to a hindered phenol group.

The hydrocarbon chain of the molecule of phenolic stabilizer improves mixing of the antioxidant with the polymeric material, such as polybutadiene, while a hindered phenol group forming a head of the molecule acts as a hydrogen donor thereby buffering oxygen from outside.

In a further embodiment, a weight ratio of the molecules of antioxidant in the first layer is at most 0.3%, preferably 0.1%.

These concentration ranges keep acoustic properties of the polymeric layer unchanged, while efficiently preventing the layer from its oxidation caused by exposure to the atmosphere.

In another embodiment of the present invention the ultrasound array further comprises at least one drive circuit coupled to the cell and adapted to (a) bring the membrane into a collapsed state in which the membrane is collapsed to the substrate, by applying a d.c. voltage over the first and the second electrodes of the at least one CMUT cell, and (b) activate the CMUT cell by applying an a.c. voltage having a CMUT operating frequency over the first and the second electrodes of the at least one said CMUT cell.

The benefit of the acoustic window layer comprising the polymeric material, which contains carbon and hydrogen atoms and has a density equal or below 0.95 g/cm$^3$ and an acoustic impedance equal or above 1.4 MRayl, is especially pronounced for the CMUT arrays having the drive circuitry adapted to operate the array in the collapsed mode. In this mode a central part of the cell membrane is brought into the contact with the substrate, which results in the biggest membrane displacement compared to the conventional operation mode. This imposes more strict requirements to the acoustic window layer properties in order to provide an improved acoustic coupling of the CMUT transducer operating in the collapsed mode. The acoustic window layer may need to adopt its inner surface to the membrane's displacement. The relatively low density of the polymeric material combined with its relatively low hardness (below 60 ShoreA, preferably below 50 ShoreA) may provide an improved acoustic contact between the acoustic window layer and the CMUT's membrane adapted to vibrate. In addition the low acoustic wave attenuation of this polymeric material may provide an improved transition of the wave throughout the acoustic layer.

In a further embodiment of the present invention the ultrasound array has a fractional bandwidth above 80%, preferably above 100%.

The ultrasound array overlaid by the first layer of the acoustic window layer is provided with optimal conditions for the acoustic wave propagation, wherein the first layer is in a direct contact with the cell membrane and comprises antioxidant molecules and the polymeric material, containing carbon and hydrogen atoms only and having a density equal or below 0.95 g/cm$^3$ and an acoustic impedance equal or above 1.4 MRayl. Thus, the ultrasound array of the present invention has an ultra-wide bandwidth due to the improved acoustic properties (stable over time) of the acoustic window layer of the present invention, compared to the conventional acoustic layers. This provides an additional advantage to the ultrasound CMUT array, capable of operating at variable ultrasound frequencies, such as in between 2 MHz

to 30 MHz.

In another embodiment of the present invention the polymeric material further comprises particles embedded therein for acoustic impedance adjustment.

The introduction of the embedded into the polymeric material particles (preferably insulating) provides a possibility of increasing a total impedance of the acoustic layer brining it closer to an ultrasonicated tissue impedance value of about 1.6 MRayl. Due to the fact that the polymeric layer exhibits such a low acoustic energy loss (attenuation), possible additional acoustic losses caused by the embedded particles may be sufficiently low in order to influence a quality of the acoustic wave propagation through the acoustic window layer. When the first layer of the acoustic window layer comprises the polymeric material with embedded insulating particles a direct acoustical coupling of the acoustical window layer to the membrane of the CMUT cell is provided. Thus, no additional coupling medium between the acoustic window and the CMUT array is required. Moreover, the acoustic impedance equal or above 1.5 MRayl matches closer to the impedance of the ultrasonicated tissue. In addition, due to the polymeric layer material's relatively low density and relatively high acoustic impedance, compared to silicon rubbers for example, a relatively smaller percentage by weight of the particles (embedded in the polymeric layer) based on the total weight of the first layer may need to be added for the further acoustic impedance adjustment.

In an embodiment of the present invention the acoustic window layer further comprises a second layer facing the outer surface and having a larger hardness than the first layer.

The additional second layer, such as polymethylpentene, being relatively harder than the first layer, may provide an improved chemical stability to the acoustic window layer against the influence of environment on the outer surface side.

Yet, in another embodiment of the present invention the acoustic impedance difference between the first layer and the second layer is smaller than 0.3 MRayl, preferably smaller than 0.1 MRayl.

This condition minimizes an acoustic impedance mismatch between the first and second layers, such that the propagating through the acoustic window layer acoustic wave does not experience a considerable backscattering at the boundary of the two layers.

Yet, in another embodiment of the present invention a method for manufacturing an ultrasound array comprising: dissolving a polymeric material in a solvent such that a liquid mixture of the polymeric material and antioxidant molecules is provided; providing a chip having at least one CMUT cell coupled to an integrated circuitry; dipping the chip in the liquid mixture or dispensing the liquid mixture on the chip, such that a layer comprising the liquid mixture overlays the CMUT cell; curing the layer at a temperature sufficient to evaporate the solvent from the liquid mixture, such that an acoustic window layer comprising a polymeric material is provided overlaying the CMUT cell.

This novel method can be scaled up to an industrial production of the acoustic window layers based on this polymeric material. Moreover, it permits creating a homogenous layer of the acoustic window material (with properties stable over time) on a chip with a CMUT cell coupled to an integrated circuitry by the dipping technique. The dipping or dispensing techniques can be beneficially used for different chip sizes comprising different ways of the CMUT bonding to the integrated circuitry, such as wire bonding, wherein the commonly used acoustic window materials coated with the commonly used manufacturing methods fail in providing a homogenous acoustic layer having a tissue matching acoustic impedance and minimized acoustic losses.

In another embodiment of the method the polymeric material, such as polybutadiene, contains hydrogen and carbon atoms and has a density equal or below 0.95 g/cm$^3$ and an acoustic impedance equal or above 1.4 MRayl.

The low attenuation characteristics of the butadiene combined with the possibility of increasing the acoustic impedance of the layer by adding the particles, preferably insulating, into the liquid mixture embedded therein lowers the strict requirements to the acoustic window layer thickness throughout the array. This provides a fast and easy method for the ultrasound array manufacturing.

These and other aspects of the invention will be apparent from and elucidated with reference to the embodiments described hereinafter.

## BRIEF DESCRIPTION OF THE DRAWINGS

In the drawings:

- FIG. 1 shows schematically and exemplarily a side view of a CMUT cell of an ultrasound array comprising an acoustic window layer having a first layer comprising antioxidant molecules and a polymeric material;
- FIG. 2 shows schematically and exemplarily a side view of a CMUT cell of an ultrasound array comprising an acoustic window layer having the first layer and a second layer facing the outer surface side and comprising a durable layer;
- FIG.3 is a graph showing a reference acoustic signal;
- FIG.4 shows a normalized output pressure of the ultrasound array comprising at least one CMUT cell and an acoustic window layer having a first layer comprising a polybutadiene;
- FIG.5 shows a normalized output pressure of the ultrasound array comprising at least one CMUT cell and an acoustic

window layer having a first layer comprising a polybutadiene and the second layer comprising polymethylpentene ;

FIG.6 is a graph comparing an acoustic frequency dependence of an acoustic loss (in dB) per millimeter for acoustic energy passing therethrough for different acoustic window materials;

FIG.7 schematically illustrates a method for manufacturing an ultrasound array in accordance with the present invention;

FIG.8 shows a photo of a chip comprising an ultrasound array together with bonding wires before (a) and after (b) the acoustic window layer deposition;

FIG.9 shows a schematic illustration of an embodiment of an ultrasound imaging system;

FIG.10 shows schematically and exemplarily a side view of a CMUT cell operating in the collapsed mode and being acoustically coupled to the acoustic window layer;

FIGs 11a-11d illustrate the principles of collapsed mode CMUT operation;

FIG. 12 illustrates the change in frequency of returning echo (ultrasound) signals as a function of time and depth,

FIG. 13 illustrates the variation of the d.c. bias voltage used to respond to the changing frequencies of returning echo signals shown in FIG.12.

DETAILED DESCRIPTION OF EMBODIMENTS

Fig. 1 shows schematically and exemplarily a CMUT cell in cross section according to the present invention. Such CMUT cell is typically fabricated on a substrate 4, such as a silicon wafer. An ultrasound array of an ultrasound system may comprise one or more CMUT cells 6. The CMUT cells may be either individually activated or in combination with each other. The individual cells can have round, rectangular, hexagon or other peripheral shapes.

Each CMUT cell has at least a pair of electrodes 7' and 7 separated by a cavity 8. The cavity 8 is formed in between a membrane 5 that is suspended over a cell floor 31 formed by the top surface of the substrate 4. The membrane 5 may be made of silicon nitride and is adapted to move or vibrate. It can be suspended over the cell floor 31 (or substrate) through a plurality of supporting portions 9 (in Fig.1 two supporting portions 9 are shown). The electrodes 7, 7' are made of electrically conductive material, such as metal. The bottom electrodes 7 may be embedded in the floor of the cell 31, while the top electrode 7' may be embedded in the membrane 5. The electrode 7 and 7' may be also deposited on the cell floor 31 or the membrane 5 as additional layers. The bottom electrode 7 is typically insulated on its cavity-facing surface with an additional layer (not pictured). This insulating layer can comprise either one of or a combination of an oxide-nitride-oxide (ONO) dielectric layer, silicon oxide layer, aluminum or hafnium oxide layers. The insulating layer may be formed above the bottom electrode 7 and below the membrane electrode 7'. The ONO-dielectric layer advantageously reduces charge accumulation on the electrodes which leads to device instability, drift and reduction in acoustic output pressure. The supporting portions 9 may be made of an insulating material such as silicon oxide or silicon nitride. The cavity 8 can be either air- or gas-filled, or wholly or partially evacuated. Two electrodes 7 and 7' separated by the cavity 8 represent a capacitance. An application of electrical signal through a drive circuit 45 coupled to the electrodes 7 and 7' causes a mechanical movement/vibration of the membrane 5, which results in the change of the capacitance and can be manipulated by an associated with the CMUT transducer integrated circuitry. The drive circuit 45 can be implemented as an integrated part of the integrated circuitry. The drive circuit 45 usually comprises an a.c. signal and a d.c. voltage sources and associated to these sources circuitry.

According to the principles of the present invention, the membrane 5 of the CMUT cell is acoustically coupled to an acoustic window layer 13 overlaying the cell membrane, and having an inner surface opposing the cell membrane and an outer surface located in the opposite direction of the inner surface. The outer surface may be either a patient or an object facing side, which can be the subjects of the ultrasound examination. The acoustic window layer comprises antioxidant and a first layer comprising a polymeric material 47. In accordance with the present invention the polymeric material contains hydrogen and carbon atoms and has a density equal or below 0.95 g/cm$^3$ and an acoustic impedance equal or above 1.4 MRayl. In particular, suitable materials may be selected from thermoset rubbers (elastomers).

Due to the limited content of the polymeric material to hydrogen and carbon atoms, the density of the material is relatively low compared to other polymeric materials, in particular rubbers.

Elastomers are generally characterized by wide-meshed crosslinking of the "knotted" molecular chains. This type of crosslinking means that the materials have a high level of dimensional stability but are still elastically malleable. By applying load (for instance tensile load) the chains become stretched, but after removal of the load they relax again. A typical hardness of the uncured elastomers is below 50 ShoreA, measured by a durometer (A scale). In general, cured (baked) elastomers exhibit higher hardness than50 ShoreA. In order to keep the hardness below 50 ShoreA the elastomer comprising layer can be under-cured (the solvent is not entirely evaporated as described in step ); in addition in order to keep the elastomer from oxidizing over time under exposure to oxygen from the atmosphere the antioxidant molecules are added to the first layer as well. This will be discussed below in detail.

Elastomers can be also thermoset, the individual molecular chains of the thermosets are characterized by three-dimensional closely meshed irreversible crosslinking. Thermoset elastomers are chemically and mechanically

more stable among elastomers, moreover in contrast to not meltable "pure" elastomers, thermoset elastomers may be processed similar to thermoplastics.

Suitable thermoset elastomers containing hydrogen and carbon atoms and having density equal or below 0.95 g/cm$^3$ (in particular in between 0.85 and 0.95 g/cm$^3$) are listed in Table 1. The chemical formula represents monomers used for the polymer chains formation. For example, the initial polymer chains of the polybutadiene and bytul rubbers comprise olefins (also referred as alkenes). During the cross-linking process of the thermoset manufacturing many double carbon-carbon bonds of the polymeric chains are broken to form a network of intrechain bonds (cross-links).

Table 1: Examples of the thermoset elastomers containing hydrogen andcarbon atoms and having a density equal or below 0.95 g/cm$^3$ at temperature of 25 degrees centigrade.

| Thermoset elastomer | Chemical formula | Density (g/cm$^3$) at 25°C |
|---|---|---|
| Polybutadiene | 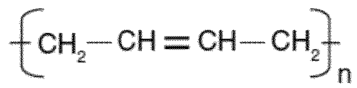 | 0.9-0.95 |
| Butyl (isobutylene-isoprene copolymer) | 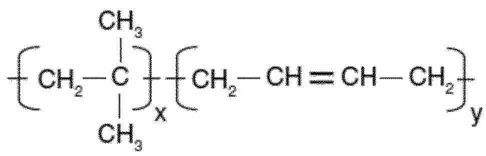 | about 0.92 |
| Ethylene propylene (EPM/ EPDM) | 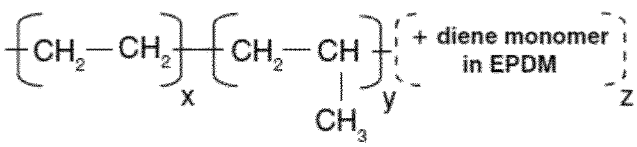 | 0.85-0.87 |
| Isoprene (synthetic cis-polyisoprene) | 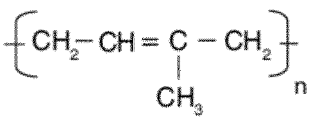 | 0.91-0.92 |
| Natural | 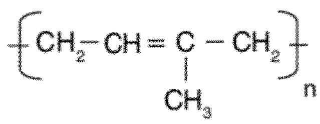 | 0.91-0.93 |

Depending on the application a thermoset elastomer with different hardness can be selected. Uncured polybutadiene rubber has a hardness of below 50 ShoreA, while butyl rubber, which polymeric chains consist of two monomers: isobutylene and isoprene, may show hardness values as low as 40 ShoreA. In order to reduce the hardness even lower, fatty acids can be added into a liquid mixture of the elastomer material. This will be discussed below in detail.

Polymeric materials having properties in accordance with the present invention showed to have a considerably lower attenuation (acoustic energy loss measured in dB per millimeter) for an acoustic wave having its frequency in range from 1 to 20 MHz. Fig.6 shows an acoustic frequency dependence of attenuation of the acoustic energy passing therethrough for different acoustic window materials. Symbols indicate measured data and lines indicate simulated dependencies. The presented materials show a steady increase of the attenuation value with the increase in frequency. The highest attenuation, which increases considerably with the frequency, is observed for the commonly used filled silicon rubber (RTV-560, curve 81): the attenuation reaches almost 5 dB/mm at a frequency around 7 MHz. The smallest attenuation is observed for polybutadiene (curve 85), which shows attenuation below 0.5 dB/mm at frequencies below 5MHz and 1 dB/mm at frequencies below 10MHz. As will be described further, even an introduction of the insulating particles embedded into the polybutadiene material does not considerably increase the attenuation of the layer. Curve 84 shows that the attenuation of the polybutadiene layer having 20% of its total weight embedded with $ZrO_2$ particles remains below 1dB/mm at frequencies below 5MHz and 2 dB/mm at frequencies below 10MHz. For comparison, the polymethylpentene material (Mitsui trade name TPX, curve 83) shows attenuation somewhat in between the polybutadiene and RTV varying from about 0.5 dB/mm at 2 MHz up to 3 dB/mm at 10 MHz. It has been shown that in order to keep acoustic properties of the first layer constant (prevent it from further hardening) antioxidant molecules are added to the first layer. A weight ratio of the molecules of antioxidant in the first layer is preferably at most 0.3% or better around

0.1%.

In accordance with the principles of the present invention the reported improvement in the acoustic wave transmission (lower attenuation) through the acoustic window layer 13 comprising the polymeric material 47, such as polybutadiene, can be attributed to the different mechanism of the electro-acoustical transformation in the CMUT compared to the PZT. The PZT-based transducer typically has a parallelepiped shape, wherein at least one of its faces is adapted to vibrate in a piston-like motion during the transmission of the acoustic wave. The displacement of the vibrating (active) face is homogeneous throughout the face surface.

In contrast, the CMUT's vibrating membrane has a different displacement throughout the membrane's area (surface). In a conventional operation mode the membrane's displacement is highest in the central part of the CMUT cell and lowest at the periphery of the membrane. In a collapsed mode of operation as shown in Fig. 10 the membrane 5 of the CMUT cell 6 is partially contacting the cell floor 31, which results in the biggest membrane displacement (D) compared to the conventional operation mode. During the CMUT operation a central part of the membrane 1 may be brought into the contact with (collapsed to) the cell floor 31 by applying a collapsed d.c. voltage value (the d.c. voltage is supplied by the drive circuitry 45). The applied a.c. signal voltage supplied by the drive circuitry 45 causes the suspended portions of the membrane 43 (located at a periphery of the membrane) to move/vibrate with an amplitude d under applied electrical signal in between electrodes 7 and 7'. From technology point of view, the CMUT with the collapsed membrane can in principle be manufactured in any conventional way, comprising providing a CMUT with a membrane and applying different means, such as electrical (bias voltage) or pressure, in order to bring the membrane to a collapsed state. In the collapsed operation mode, the displacement D of the central part of the membrane is fixed, while the suspended portions of the membrane vibrate with an amplitude d, which is determined by the applied a.c. voltage signal.

The variation in the displacement of the membrane's vibrating portions imposes different requirements on the acoustic window layer properties in order to provide an improved acoustic coupling of the operating CMUT transducer. The acoustic window layer may need to adopt its inner surface to the membrane's displacement. The relatively light molecular weight of the monomers of the polymeric material 47, preferably thermoset elastomer, combined with the material's relatively low hardness (below 60 ShoreA, preferably below 50 ShoreA ) may provide an improved acoustic contact between the acoustic window layer 13 and the CMUT's membrane adapted to vibrate. In addition the low acoustic wave attenuation of this polymeric material may provide an improved transition of the wave throughout the acoustic layer 13.

It has been found that in the absence of antioxidant molecules acoustic layer comprising polybutadiene increases hardness of the polymeric material caused by aging due to its oxidation. When the hardness of the acoustic layer being in contact with the CMUT array increases above 60 Shore the layers acoustic transmission properties (for the CMUT array) are substantially reduced. It is undesirable to have an acoustic material having its properties changing with time, further it is even more undesirable to end up with an acoustic window layer having unsuitable acoustic wave transmission.

In accordance to the present invention antioxidant molecules are added to the polybutadiene layer. For example, molecules of phenolic stabilizer can be used. Phenolic stabilizers are primary antioxidants that act as hydrogen donors in the polymeric layer. They react with peroxy radicals to form hydroperoxides and prevent the abstraction of hydrogen from the polymer backbone.

In the present embodiment, sterically hindered phenol was used with uncured polybutadiene layer. The molecule of this antioxidant (BASF trade name Irganox 1076) has a hydrocarbon chain (or tail) coupled to a hindered phenol group (head).

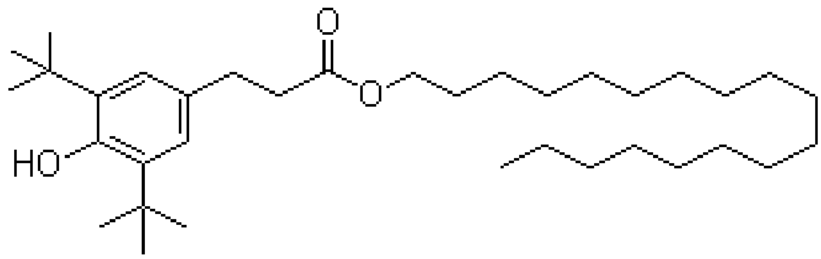

Phenolic Primary Antioxidant: Irganox 1076

The hydrocarbon chain of the molecule of phenolic stabilizer improves mixing of the antioxidant with the polymeric material, such as polybutadiene, while a hindered phenol group forming a head of the molecule acts as a hydrogen donor thereby buffering oxygen from outside.

Another embodiment of the present invention is illustrated in Fig.2. The acoustic window layer 13 may further have a second layer comprising a durable exterior layer 42, which is located at the outer surface side. The durable

exterior layer located as the outer surface may satisfy different mechanical and/or chemical characteristics of the acoustic window layer 13. For example, depending on the purpose of the ultrasound transducer, the following material may be used in the second layer: materials having a different abrasion resistance to the polymeric material 47, material with different friction coefficients. In order to improve the chemical stability of the acoustic window the second layer may have a layer of thermoplastic polymer, such as polymethylpentene; and in order to provide moisture barrier layers of mylar, polyethylene and/ or parylene may be introduced into the second layer.

In order to vary a total hardness of the acoustic window layer 13, an additional layer of a polyolefin thermoplastic elastomer may be selected. For example, copolymers having monomers of ethylene and alpha olefins, such as octane or butane. Thermoplastic elastomers (TPE), sometimes referred to as thermoplastic rubbers, are a class of copolymers or a physical mix of polymers (usually a plastic and a rubber) which consist of materials with both thermoplastic and elastomeric properties.

It shall be understood by a person skilled in the art that additional layers providing the acoustic window layer with improved biocompatibility or elasticity properties may be introduced into the acoustic window 13.

Fig.3 illustrates a reference acoustic signal transmitted by the CMUT cells of the array through the acoustic window layer. Fig.4 illustrates a normalized output pressure 63 of the array overlaid by a 30 micrometer thick polybutadiene layer capped with the second layer of 5 micrometer thick parylene. As can be seen in Fig.4 the polybutadiene layer provides superior acoustic properties to the acoustic window, namely ultrawide band-width and high sensitivity. Intersection points 62 of a horizontal line 61with the output pressure curve 63 of the array give a lower cut-off frequency ( $f_1$) of about 5MHz and an upper cut-off frequency ( $f_2$) of about 27 MHz at the signal attenuation of 3 dB. Thus, the bandwidth of the array is about 140 %. Note, that the reference is made to a fractional bandwidth (FBW%) calculated as percentage value of

$$FBW=2\cdot(f_2-f_1)/(f_2+f_1).$$

A relative sensitivity of this array is about 4 MPa per 100 V of the applied a.c. signal. The relative sensitivity is determined in the following way, for each given a.c. signal applied in between the CMUT electrodes 7,7' (for instance 20V) an output pressure is measured and then normalized to the output pressure achieved at 100V.

Fig.5 illustrates a normalized output pressure 64 of the array overlaid by 60 micrometer thick polybutadiene layer capped with the second layer of TPX material, wherein the TPX layer is 0.2 millimeter thick and is glued to the first layer of polybutadiene with a third layer of polyurethane. Intersection points 66 of a horizontal line 65 with the output pressure curve 64 of the array give a lower cut-off frequency ( $f_1$) of about 5MHz and an upper cut-off frequency ( $f_2$) of about 23 MHz at the signal attenuation of 3 dB. Thus, the bandwidth of the array is about 130 %. Even the introduction of the durable material layer that is thicker than the first layer of polybutadiene interfacing with the CMUT membrane does not considerably reduce the ultrawide band-width of the array. The sensitivity of the array in this embodiment is about 3 MPa per 100 V. An additional advantage of the TPX material is its chemical stability, especially towards commonly used cleaning agents in medical applications, and mechanical resistance.

The polymeric layer, especially thermoset elastomers, which contain hydrogen and carbon atoms and have density equal or below 0.95 g/cm$^3$ may exhibit a low acoustic energy loss (attenuation) and a suitable acoustic impedance optimization.

Acoustic impedance (Z) is defined as the product of acoustic propagation velocity (v) for acoustic energy (or wave) in a medium and density (p) of this medium:

$$Z=\rho*v.$$

These polymeric materials may have an acoustic impedance value of above 1.4 MRayl, which is closer to a soft tissue's impedance of about 1.6 MRayl. In order to minimize an impedance mismatch between the ultrasound array and the ultrasonicated tissue it may be desirable to increase the acoustic impedance value of the acoustic window material comprising the polymeric layer. This can be achieved by adding a filler, such as particles 41 (preferably insulating), into the polymeric layer 47. The introduction of the particles into the polymeric layer increases a total density of the first layer. It has been discovered that the additional acoustic losses caused by the embedded insulating particles are sufficiently low and do not considerably influence a quality of the acoustic wave propagation through the polymeric layer.

As an example, Table 2 shows the measured changes in acoustic properties of the polybutadiene layer with the introduction of zirconium dioxide ($ZrO_2$) insulating particles having in average diameter of about 2.5 micrometer and taking a fixed percentage of a total weight of the first layer.

Table 2: Changes in the density, acoustic wave velocity, acoustic impedance and attenuation (at frequency of 7 MHz) with an increasing percentage by weight of $ZrO_2$ particles based on the total weight of the polybutadiene layer.

| % ZrO2 | Density (g/cm$^3$) | Velocity (mm/ microsec) | Impedance (MRayl) | Attenuation @7 MHz (dB/mm) |
|---|---|---|---|---|
| 0 | 0.906 | 1.570 | 1.423 | 0.55 |
| 4% | 0.937 | 1.553 | 1.455 | 0.75 |
| 8% | 0.972 | 1.532 | 1.489 | 0.87 |
| 16% | 1.0405 | 1.503 | 1.564 | 1.05 |
| 20% | 1.0855 | 1.469 | 1.5945 | 1.25 |

As can be seen from the table with the total density increase of the first layer comprising polybutadiene, the acoustic impedance of the layer can be tuned towards higher values, e.g. closer to the tissue's acoustic impedance, while the attenuation of the layer still remains below 1.5 dB/mm, even for the layers comprising 20% of its weight taken by the insulating particles (ZrO2).

When the first layer of the acoustic window layer, comprising the antioxidant molecules and the polymeric material, which contains hydrogen and carbon atoms and has a density equal or above 0.90 g/cm$^3$ and an acoustic impedance equal or above 1.4 MRayl, a direct acoustical coupling of the acoustic window layer to the membrane of the CMUT cell is provided. Thus, no additional coupling medium between the acoustic window and the CMUT array is required.

The additional advantage of the polymeric materials containing hydrogen and carbon atoms and having a density equal or below 0.95 g/cm$^3$ and an acoustic impedance equal or above 1.4 MRayl, preferably 1.5 MRayl, is that compared to commonly used silicon based rubbers (with a typical acoustic impedance value of round 1.1-1.2 MRayl) in ultrasound, these polymeric materials, in particular polybutadiene, possess higher acoustic impedance. Therefore, in order to tune the acoustic impedance of the polymeric layer 47 to a tissue's impedance a relatively smaller amount of the filler may be used in this polymeric material, compared to the filled silicones. Since an introduction of the insulating particles to a layer on average increases its hardness, an application of these polymeric materials having higher acoustic impedance provides the acoustic window layer 13 with relatively smaller changed hardness after filling (remaining below 60 ShoreA, preferably below 50 ShoreA) and a considerably lower attenuation (preferably below 1.5 or 2 dB/mm) compared to filled silicones. In order to bring the acoustic impedance of the filled silicon closer to the soft tissue's impedance, i.e. from 1.1 MRayl to 1.6 MRayl, a larger amount of the filler particles is required. This introduction of the particles introduces considerable attenuation and increases the hardness of the filled silicon layer.

An optimum acoustical coupling of the acoustic window layer to the membrane of the CMUT cell can be provided by a combination of uncured polymeric material having low attenuation and acoustic impedance above 1.4 MRayl, said material being resistant to oxidation over time (aging or degradation); and a relatively constant hardness of the first layer with an introduction of the embedded insulating particles.

An advantage of the application of the acoustic window layer comprising the first layer in accordance with the present invention becomes even more pronounced for the transducer arrays using CMUT cells operating in the collapsed mode. The collapsed mode of operation allows operating an ultrasound array in a wide range of acoustic waves frequencies, which can be tuned by varying a dc-bias voltage applied to the CMUTs electrodes.

Thus, imposing higher requirements on the acoustic window layer bandwidth, compared to a conventional (non-collapsed) CMUT operation mode. The preferred bandwidth for the CMUT ultrasound array overlaid by the acoustic window layer 13 in accordance with the principles of the present invention is above 80%, preferably above 100% or 120%. A different from the PZTs electrical to acoustic energy conversion principle used in the CMUTs makes CMUT arrays more demanding towards the acoustic window layers being in the direct contact with the CMUTs vibrating membranes, which may become even more pronounced for the collapsed mode of the CMUT operation.

The frequency response of the CMUT in the collapsed mode is varied by adjusting the d.c.bias voltage applied to the CMUT electrodes (7.7') after collapse. As a result, the resonant frequency of the CMUT cell increases as higher DC bias is applied to the electrodes. The principles behind this phenomenon are illustrated in Figs 11a-11d. The cross-sectional views of Figs 3a and 3c illustrate this one dimensionally by the distances $D_1$ and $D_2$ between the supporting portions 9 of the membrane 5 and the point where the membrane begins to touch the floor 31 of the cavity 8 in each illustration. It can be seen that the distance $D_1$ is a relatively long distance in Fig. 11a when a relatively low bias voltage is applied after collapse, and the distance $D_2$ in Fig. 11c is a much shorter distance when a higher bias voltage is applied. These distances can be analogized to long and short strings which are held by the ends and then plucked. The long, relaxed string will vibrate at a much lower frequency when plucked than will the shorter, tighter string. Analogously, the

resonant frequency of the CMUT cell in Fig. 11a will be lower than the resonant frequency of the CMUT cell in Fig. 11c which is subject to the higher d.c. pulldown bias voltage.

The phenomenon can also be appreciated from the two dimensional illustrations of Figs. 11b and 1 Id, as it is in actuality a function of the effective operating area of the CMUT membrane. When the membrane 5 just touches the floor of the CMUT cell as shown in Fig. 11a, the effective vibrating area $A_1$ of the noncontacting (free vibrating) portion of the cell membrane 5 is large as shown in Fig. 3b. The small hole in the center 17 represents the center contact region of the membrane. The large area membrane will vibrate at a relatively low frequency. This area 17 is an area of the membrane 5, which is collapsed to the floor of the CMUT cell. But when the membrane is pulled into deeper collapse by a higher bias voltage as in Fig. 11c, the greater central contact area 17' results in a lesser free vibrating area $A_2$ as shown in Fig. 11d. This lesser area $A_2$ will vibrate at a higher frequency than the larger $A_1$ area. Thus, as the d.c. bias voltage is decreased the frequency response of the collapsed CMUT cell decreases, and when the d.c. bias voltage increases the frequency response of the collapsed CMUT cell increases.

Therefore, it is desirable providing an optimal acoustic window layer 13, which permits fully exploring a broad frequency band, in the CMUT based ultrasound array can operate. Preferably, the acoustic window layer exhibits the acoustic loss per millimeter for acoustic energy passing therethrough of less than 3 dB, preferably 1.5 dB, for a wide range of the acoustic wave frequencies applicable in medical ultrasound, such as in between 2 and 30 MHz or 5 and 25 MHz.

A broad bandwidth of the CMUT array can be used, for example, by continually varying the CMUT's frequency during echo (reflected in the body ultrasound waves) reception. Fig. 12 illustrates the progressive decline in the center frequency of echo signals 52, 54, 56 as the echoes are received from increasing depths over time as shown by the ordinate axis of the illustration. The line 55 plots the steady decline in center frequency with depth (time). As echoes are received from shallow depths and then from progressively deeper depths, the d.c. bias voltage of a collapsed mode CMUT is varied from a higher voltage to a lower voltage as shown by the line 50 in Fig. 13, and the center frequency of the CMUT cells declines correspondingly. The frequency response of the collapsed mode CMUT array is continually tailored to follow the depth-dependent frequency attention by this method of d.c. bias control.

To have a better control over the attenuation properties of the first layer 47 the insulating particles may have an average size smaller than one tens of an acoustic wavelength. Preferably the average size of the particles is lower than one tens of the acoustic wavelength of the shortest wave within an operation bandwidth of the ultrasound array. The bandwidth may be selected based on the ultrasound application such as high frequency (above 15 MHz) and high image resolution cardio-imaging. When the average size of the particles becomes larger than the wavelength of the propagating wave, an additional scattering in the acoustic window layer may happen. This may introduce artifacts in an ultrasound image.

Particular average sizes of the particles may be: in between 10 nanometer and 10 micrometer; in between 10 nanometer and 100 nanometer; or in between 1 micrometer and 10 micrometer. These ranges of the average particles cover main application of the ultrasound imaging systems at different frequencies. For example, for acoustic wave velocity of 1500m/s (meter) one tens of the acoustic wavelength is: 150 micrometer for frequency of 1MHz; 15 micrometer for frequency of 10MHz and 5 micrometer for frequency of 30MHz.

In an embodiment of the present invention it may be beneficial in using ceramic particles as the insulating particles. Ceramic particles, such as metal oxides (ZrO2, Al2O3, TiO2, Bi2O3, BaSO4, etc.) show high insulating properties, which may be advantages in providing additional insulation to the arrays electronics. Moreover, there multiple ways developed in the art for manufacturing ceramic particles of a well-defined size.

A developed industrial application of the polymeric materials is described further. In Fig.7 a novel method 20 for manufacturing an ultrasound array in accordance with the present invention is illustrated. A granulate of pre-polymerized polybutadiene 38 (CB728 T from Lanxess) is provided in step 31. In step 32 the block is granulated and dissolved in solvents like alkanes, branched or cyclic alkanes, for example hexane, heptane, cyclohexane. In an optional step 33 antioxidants such as sterically hindered phenol (Iraganox 1076 in this example) is added, further the optimization of the acoustic impedance of the first layer can be achieved by adding insulating particles to the solvent, wherein the polymeric material act as a dispersion agent for the particles, such that a liquid mixture of the polymeric material and the insulating particles is provided. The additional dispersion agents like fatty acids (a carboxylic acid with an aliphatic chain, which is either saturated or unsaturated) may be added in the liquid mixture. The filler particles in the liquid mixture may increase the hardness of the first layer of the acoustic window, while fatty acids may counter play this hardness increase keeping the average hardness of the first layer at the relatively constant value. The unsaturated chains of fatty acid like oleic acid, linoleic acid and linolenic acid (one, two and respectively tree double carbon bonds) can polymerise and bond to the polybutadiene chains. This provides a good dispersion/distribution of particles in the liquid mixture. In step 54 a chip having the ultrasound array with at least one CMUT cell coupled to an integrated circuitry is provided. In step 34 the chip is dipped in the liquid mixture, such that a layer comprising the liquid mixture overlays the CMUT cell. As an alternative in the step 34, the liquid mixture can be dispensed over the chip using the known to the skilled person dispersion techniques. Since in step 33 a minimum impedance mismatch between the liquid mixture and the propagating medium

can be achieved, the tolerance to the thickness variation of the liquid mixture layer is rather high. The increase in the dipping time would increase the thickness of the liquid mixture layer. In the step 35 the chip with the liquid mixture layer may be let drying at elevated temperature of about 70°C. With the time, when the solvent starts evaporating from the liquid mixture, the liquid mixture layer may become more solid (sticky). At this stage the second layer of another material can be applied to the liquid mixture layer. The advantage of this step is that the second layer can be attached to the first layer without any glue. In step 36 the layer overlaying the CMUT cell is cured at a temperature sufficient to evaporate the remaining solvent (about 100°C, in the case of heptane) from the liquid mixture layer, such that an acoustic window layer 13 comprising a polymeric material 47 with insulating particles 41 embedded therein is provided overlaying the CMUT cell. Alternately, to assure a better fixation the second layer may be coupled to the first layer with the third layer of glue and further cured in step 37.

This method can be advantageously applied on industrial scale owing to the simplicity of the steps and large tolerance of the ultrasound array's performance to the acoustic layer window thickness. The layer thickness can be increased by repeating the steps of dipping 34 and drying 35. Due to the possibility of the impedance optimization in step 33 and low attenuation properties of the selected polymeric materials in accordance to the principles of the present invention, a local thickness deviation in the acoustic window layer from the average value can be higher than the accepted standard in commonly used spray or spin coat manufacturing. In addition to this manufacturing method provides flexibility in different chip designs and electrical contact bonding implemented in the array.

For example, Fig.8 shows a photo of a chip comprising an ultrasound array 23 together with bonding wires 22 before (a) and after (b) the acoustic window layer deposition using the method of the present invention. Commonly used spray or spin-coat manufacturing methods of the acoustic lenses (or window layers) are highly layer thickness sensitive and may fail in providing a homogeneous coverage of the array around the free standing bonding wires 22 (Fig.8a shows a close up of the bonding wires 22). As can be seen in Fig.8(b) the presented method overcomes this problem providing a fully overlaid CMUT transducer array with stable acoustic window having an improved wide bandwidth performance.

The method can be also beneficially used for different chip size, especially in the area miniaturized ultrasound arrays, such as interventional devices and catheters.

FIG. 9 illustrates the principle design of an ultrasonic imaging system 202.

The ultrasound imaging system is generally denoted with reference numeral 202. The ultrasound imaging system 202 is used for scanning an area or volume of the body, e.g. of a patient 201. It is to be understood that the ultrasound system 202 may also be used for scanning other areas or volumes, e.g. body parts of animals or other living beings.

For scanning the patient 201, an ultrasound probe 200 may be provided. In the embodiment shown, the ultrasound probe 200 is connected to a console device 203. The console device 203 is shown in FIG. 9 as a mobile console. This console 203 may, however, also be realized as a stationary device. The console device 203 is connected to the probe 200 via an interface 206 formed in a wired manner. Further, it is contemplated that the console device 203 may also be connected to the probe 200 in a wireless manner, for example using UWB transmission technology. The console device 203 may further comprise an input device 205. The input device may have buttons, a key pad and/or a touchscreen to provide an input mechanism to a user of the ultrasound imaging system 202. Additionally or alternatively, other mechanisms may be present in the input device 205 to enable a user to control the ultrasound imaging system 202.

Further, the console device 203 comprises a display 204 to display display data generated by the ultrasound imaging system 202 to the user. By this, the volume within the patient 201 that is scanned via the ultrasound probe 200 can be viewed on the console device 203 by the user of the ultrasound system 200.

The ultrasound probe 200 comprises the CMUT transducer array constructed in accordance with the present invention.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems. The scope of protection is defined by the appended claims.

## Claims

1. An ultrasound array for acoustic wave transmission comprising at least one capacitive micro-machined ultrasound transducer, CMUT, cell (6), wherein the CMUT cell comprises a substrate (4); a first electrode (7); a cell membrane (5) opposing the substrate (4) and having a second electrode (7'), which opposes the first electrode with a cavity (8) there between, wherein the membrane is arranged to vibrate upon the cell activation; and

   an acoustic window layer (13), overlaying the cell membrane thereby being in direct contact with the cell membrane (5), and having an inner surface opposing the cell membrane (5) and an outer surface;
   wherein the acoustic window layer (13) comprises a first layer comprising molecules of antioxidant and a polymeric material (47), and wherein the polymeric material consists of hydrogen and carbon atoms, and wherein said first layer has a density equal or below 0.95 g/cm$^3$ and an acoustic impedance equal or above 1.4 MRayl.

2. The ultrasound array according to claim 1, wherein the polymeric material is a thermoset elastomer.

3. The ultrasound array according to claim 1 or 2, wherein the molecules of antioxidant are primary antioxidants.

4. The ultrasound array according to claim 2, wherein the thermoset elastomer is polybutadiene.

5. The ultrasound array according to claim 4, wherein the molecules of antioxidant are primary antioxidants..

6. The ultrasound array according to claim 3 or 5, wherein the molecules of antioxidant are phenolic stabilizers.

7. The ultrasound array according to claim 3 or 5, wherein a weight ratio of the molecules of antioxidant in the first layer is at most 0.3%, preferably 0.1%.

8. The ultrasound array according to claim 6, wherein each molecule of the phenolic stabilizer has a hydrocarbon chain coupled to a hindered phenol group.

9. The ultrasound array according to claim 1 or 4, further comprising at least one drive circuit (45) coupled to the cell and adapted to (a) bring the membrane (5) into a collapsed state in which the membrane is collapsed to the substrate (4) , by applying a d.c. voltage over the first (7) and the second electrodes (7') of the at least one CMUT cell, and (b) activate the CMUT cell by applying an a.c. voltage having a CMUT operating frequency over the first and the second electrodes of the at least one said CMUT cell.

10. The ultrasound array according to claim 1 or 4, wherein the first layer has a hardness below 50 Shore A.

11. The ultrasound array according to claim 1, wherein the acoustic window layer further comprises a second layer (42) facing the outer surface and having a larger hardness than the first layer.

12. The ultrasound array according to claim 11, wherein the acoustic impedance difference between the first layer and the second layer is smaller than 0.3 MRayl, preferably smaller than 0.1 MRayl.

13. A method of manufacturing an ultrasound array (20) according to claim 1, the method comprising:

   - dissolving (32) a polymeric material (38) with antioxidant in a solvent, such that a liquid mixture of the polymeric material is provided;
   - providing a chip having at least one CMUT cell coupled to an integrated circuitry;
   - dipping (34) the chip in the liquid mixture or dispensing (34) the liquid mixture on the chip, such that a layer comprising the liquid mixture overlays the CMUT cell in a direct with a membrane of the CMUT cell;
   - curing (36) the layer at a temperature sufficient to evaporate the solvent from the liquid mixture, such that an acoustic window layer (13) comprising a polymeric material (47) is provided overlaying the CMUT cell.

14. The method according to claim 13 of manufacturing an ultrasound array according to claim 9, wherein the polymeric material, such as polybutadiene, contains hydrogen and carbon atoms and has a density equal or below 0.95 g/cm$^3$ and an acoustic impedance equal or above 1.4 MRayl.

15. An interventional device comprising an ultrasound array according to any of claims 1 to 12.

**Patentansprüche**

1. Ultraschall-Array zur Übertragung akustischer Wellen, das mindestens einen kapazitiven mikrobearbeiteten Ultraschallwandler, CMUT-Zelle (6) umfasst, wobei die CMUT-Zelle ein Substrat (4) eine erste Elektrode (7); eine Zellmembran (5), die dem Substrat (4) gegenüberliegt und eine zweite Elektrode (7') aufweist, die der ersten Elektrode mit einem Hohlraum (8) dazwischen gegenüberliegt, wobei die Membran so angeordnet ist, dass sie bei der Zellaktivierung schwingt; und eine akustische Fensterschicht (13), die über der Zellmembran liegt, wodurch sie in direktem Kontakt mit der Zellmembran (5) steht, und die eine innere Oberfläche, die der Zellmembran (5) gegenüberliegt, und eine äußere Oberfläche aufweist; wobei die akustische Fensterschicht (13) eine erste Schicht umfasst, die Moleküle eines Antioxidans und ein polymeres Material (47) umfasst, und wobei das polymere Material aus Wasserstoff- und Kohlenstoffatomen besteht, und wobei die erste Schicht eine Dichte gleich oder unter 0.95 g/cm$^3$ und eine akustische Impedanz gleich oder über 1.4 MRayl aufweist.

2. Ultraschall-Array nach Anspruch 1, wobei das polymere Material ein duroplastisches Elastomer ist.

3. Ultraschall-Array nach Anspruch 1 oder 2, wobei die Moleküle des Antioxidantien primäre Antioxidantien sind.

4. Die Ultraschallanordnung nach Anspruch 2, wobei das duroplastische Elastomer Polybutadien ist.

5. Ultraschallanordnung nach Anspruch 4, wobei die Moleküle des Antioxidantien primäre Antioxidantien sind.

6. Ultraschall-Array nach Anspruch 3 oder 5, wobei die Moleküle des Antioxidantien phenolische Stabilisatoren sind.

7. Ultraschallanordnung nach Anspruch 3 oder 5, wobei ein Gewichtsverhältnis der Moleküle des Antioxidationsmittels in der ersten Schicht höchstens 0.3 %, vorzugsweise 0.1 % beträgt.

8. Ultraschall-Array nach Anspruch 6, wobei jedes Molekül des phenolischen Stabilisators eine Kohlenwasserstoffkette aufweist, die an eine geschwächte Phenolgruppe gekoppelt ist.

9. Ultraschall-Array nach Anspruch 1 oder 4, ferner umfassend mindestens eine Treiberschaltung (45), die mit der Zelle gekoppelt und geeignet ist, (a) die Membran (5) in einen kollabierten Zustand zu bringen, in dem die Membran auf das Substrat (4) kollabiert ist, indem eine Gleichspannung über die erste (7) und die zweite Elektrode (7') der mindestens einen CMUT-Zelle angelegt wird, und (b) die CMUT-Zelle durch Anlegen einer Wechselspannung mit einer CMUT-Betriebsfrequenz über die erste und die zweite Elektrode der mindestens einen CMUT-Zelle zu aktivieren.

10. Das Ultraschall-Array nach Anspruch 1 oder 4, wobei die erste Schicht eine Härte unter 50 Shore A aufweist.

11. Ultraschall-Array nach Anspruch 1, wobei die akustische Fensterschicht noch eine zweite Schicht (42) umfasst, die der Außenfläche zugewandt ist und eine größere Härte als die erste Schicht aufweist.

12. Ultraschall-Array nach Anspruch 11, wobei die akustische Impedanz-Differenz zwischen der ersten Schicht und der zweiten Schicht kleiner als 0.3 MRayl, vorzugsweise kleiner als 0.1 MRayl ist.

13. Verfahren zur Herstellung eines Ultraschall-Arrays (20) nach Anspruch 1, dabei umfasst das Verfahren:

   - Auflösen (32) eines polymeren Materials (38) mit einem Antioxidationsmittel in einem Lösungsmittel, so dass eine flüssige Mischung des polymeren Materials bereitgestellt wird;
   - Bereitstellen eines Chips mit mindestens einer CMUT-Zelle, die mit einer integrierten Schaltung verbunden ist;
   - Eintauchen (34) des Chips in die flüssige Mischung oder Auftragen (34) der flüssigen Mischung auf den Chip, so dass eine Schicht, die die flüssige Mischung umfasst, die CMUT-Zelle direkt mit einer Membran der CMUT-Zelle überlagert;
   - Aushärten (36) der Schicht bei einer Temperatur, die ausreicht, damit Lösungsmittel aus dem Chip zu verdampft die Flüssigkeitsmischung, so dass eine akustische Fensterschicht (13), die ein Polymermaterial (47) umfasst, über der CMUT-Zelle bereitgestellt wird.

14. Verfahren nach Anspruch 13 zur Herstellung eines Ultraschall-Arrays nach Anspruch 9, wobei das polymere Material, wie z.B. Polybutadien, Wasserstoff- und Kohlenstoffatome enthält und eine Dichte gleich oder unter 0.95 g/cm$^3$ und

eine akustische Impedanz gleich oder über 1.4 MRayl aufweist.

**15.** Eingriffsvorrichtung mit einem Ultraschall-Array nach einem der Ansprüche 1 bis 12.

## Revendications

**1.** Un réseau d'ultrasons pour la transmission d'ondes acoustiques comprenant au moins un transducteur ultrasonore capacitif micro-usiné, CMUT, cellule (6), dans lequel la cellule CMUT comprend un substrat (4) une première électrode (7), une membrane cellulaire (5) opposée au substrat (4) et ayant une seconde électrode (7'), qui est opposée à la première électrode avec une cavité (8) entre elles, dans laquelle la membrane est disposée pour vibrer lors de l'activation de la cellule; et
une couche de fenêtre acoustique (13), recouvrant la membrane cellulaire, étant ainsi en contact direct avec la membrane cellulaire (5), et ayant une surface intérieure opposée à la membrane cellulaire (5) et une surface extérieure ; dans laquelle la couche de fenêtre acoustique (13) comprend une première couche comprenant des molécules d'antioxydant et un matériau polymère (47), et dans laquelle le matériau polymère consiste en atomes d'hydrogène et de carbone, et dans laquelle ladite première couche a une densité égale ou inférieure à 0.95 $g/cm^3$ et une impédance acoustique égale ou supérieure à 1.4 MRayl.

**2.** Réseau d'ultrasons selon la revendication 1, dans lequel le matériau polymère est un élastomère thermodurci.

**3.** Réseau d'ultrasons selon la revendication 1 ou 2, dans lequel les molécules d'antioxydant sont des antioxydants primaires.

**4.** Réseau à ultrasons selon la revendication 2, dans lequel l'élastomère thermodurci est du polybutadiène.

**5.** Réseau d'ultrasons selon la revendication 4, dans lequel les molécules d'antioxydant sont des antioxydants primaires.

**6.** Réseau d'ultrasons selon la revendication 3 ou 5, dans lequel les molécules d'antioxydant sont des stabilisateurs phénoliques.

**7.** Réseau d'ultrasons selon la revendication 3 ou 5, dans lequel un rapport pondéral des molécules d'antioxydant dans la première couche est au maximum de 0.3 %, de préférence de 0.1%.

**8.** Réseau d'ultrasons selon la revendication 6, dans lequel chaque molécule du stabilisateur phénolique possède une chaîne hydrocarbonée couplée à un groupe phénolique encombré.

**9.** Réseau d'ultrasons selon la revendication 1 ou 4, comprenant en outre au moins un circuit de commande (45) couplé à la cellule et adapté pour (a) amener la membrane (5) dans un état replié dans lequel la membrane est repliée sur le substrat (4), en appliquant une tension continue sur la première (7) et la seconde (7') électrodes de la au moins une cellule CMUT, et (b) activer la cellule CMUT en appliquant une tension alternative ayant une fréquence de fonctionnement CMUT sur la première et la seconde électrodes de la au moins une dite cellule CMUT.

**10.** Réseau d'ultrasons selon la revendication 1 ou 4, dans lequel la première couche a une dureté inférieure à 50 Shore A.

**11.** Réseau d'ultrasons selon la revendication 1, dans lequel la couche de fenêtre acoustique comprend en outre une deuxième couche (42) faisant face à la surface extérieure et ayant une dureté plus grande que la première couche.

**12.** Réseau d'ultrasons selon la revendication 11, dans lequel la différence d'impédance acoustique entre la première couche et la deuxième couche est inférieure à 0.3 MRayl, de préférence inférieure à 0.1 MRayl.

**13.** Procédé de fabrication d'un réseau d'ultrasons (20) selon la revendication 1, le procédé comprenant:

- dissoudre (32) un matériau polymère (38) avec un antioxydant dans un solvant, de telle sorte qu'un mélange liquide du matériau polymère soit fourni;
- fournir une puce ayant au moins une cellule CMUT couplée à un circuit intégré;
- tremper (34) la puce dans le mélange liquide ou distribution (34) du mélange liquide sur la puce, de sorte

qu'une couche comprenant le mélange liquide recouvre la cellule CMUT dans un contact direct avec une membrane de la cellule CMUT;
- durcir (36) la couche à une température suffisante pour faire évaporer le solvant du mélange liquide, de telle sorte qu'une couche de fenêtre acoustique (13) comprenant un matériau polymère (47) est prévue pour recouvrir la cellule CMUT.

**14.** Procédé selon la revendication 13, consistant à fabriquer un réseau d'ultrasons selon la revendication 9, dans lequel le matériau polymère, tel que le polybutadiène, contient des atomes d'hydrogène et de carbone et présente une densité égale ou inférieure à 0.95 g/$cm^3$ et une impédance acoustique égale ou supérieure à 1.4 MRayl.

**15.** Dispositif d'intervention comprenant un réseau d'ultrasons selon l'une quelconque des revendications 1 à 12.

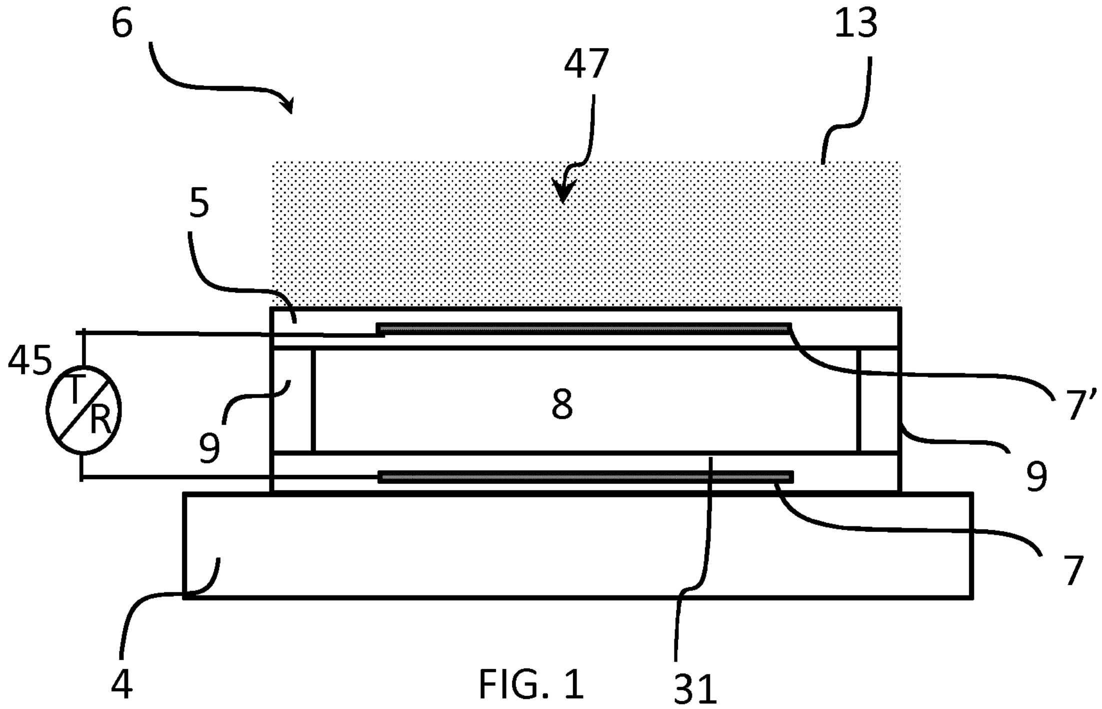


FIG. 1

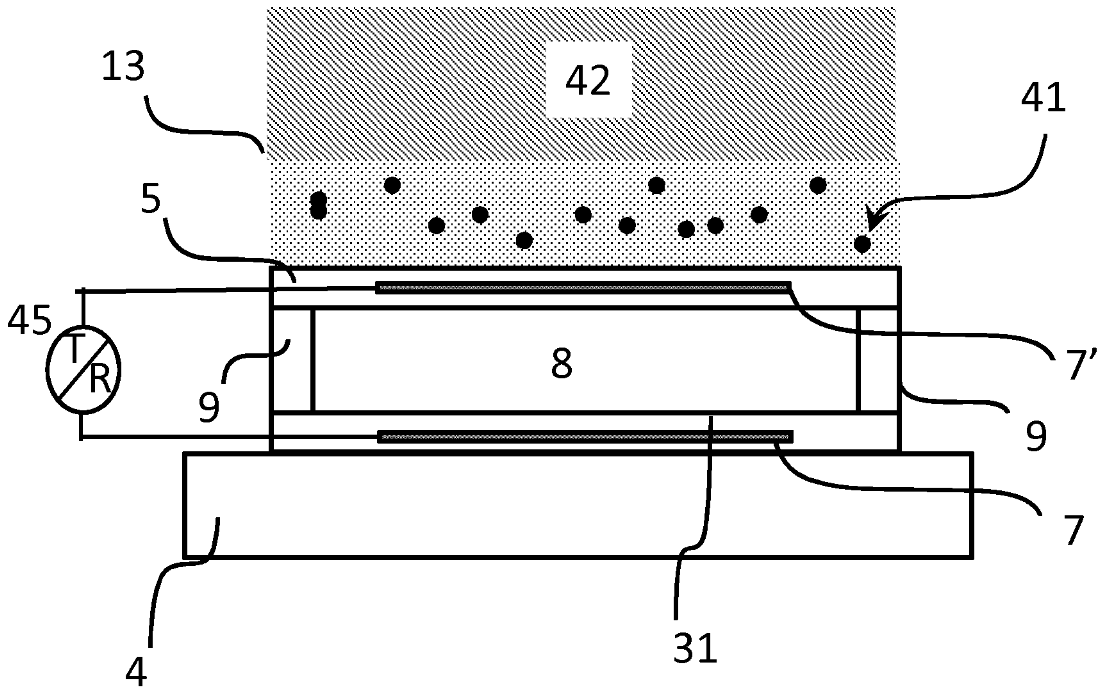


FIG. 2

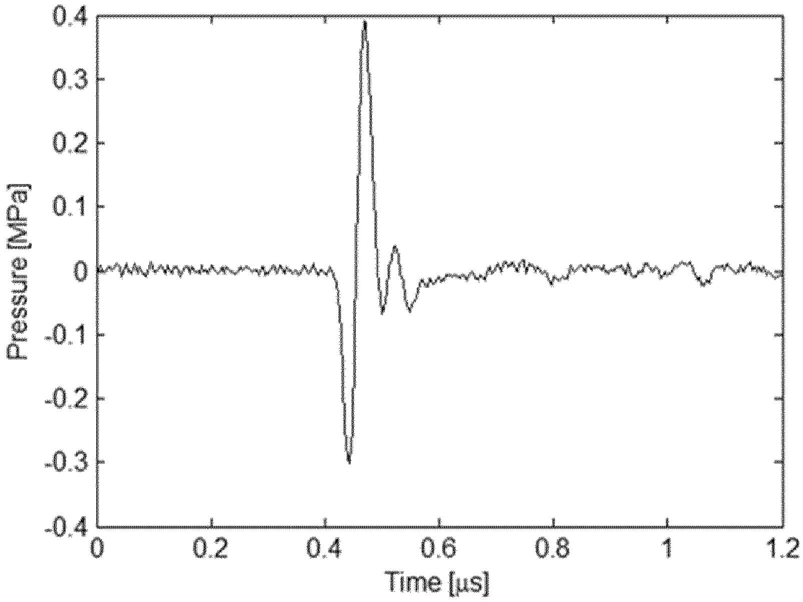
Pressure [MPa]
Time [µs]
0.4
0.3
0.2
0.1
0
-0.1
-0.2
-0.3
-0.4
0
0.2
0.4
0.6
0.8
1
1.2

FIG. 3

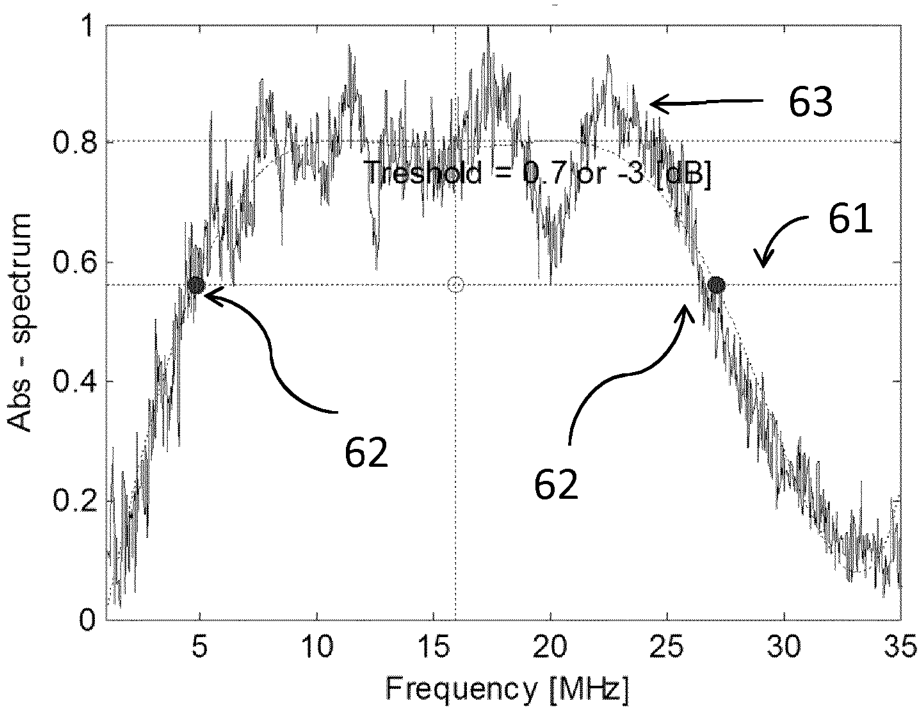
Abs - spectrum
Frequency [MHz]
Treshold = 0.7 or -3 [dB]
63
61
62
62
1
0.8
0.6
0.4
0.2
0
5
10
15
20
25
30
35

FIG. 4

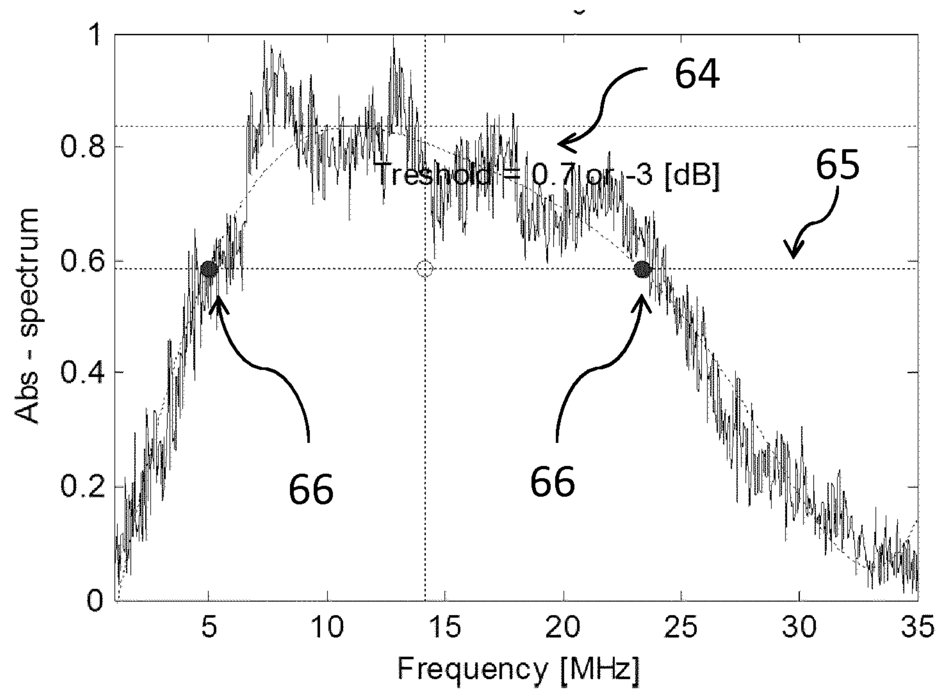

Abs - spectrum
Frequency [MHz]
Treshold = 0.7 or -3 [dB]
64
65
66
66


FIG. 5

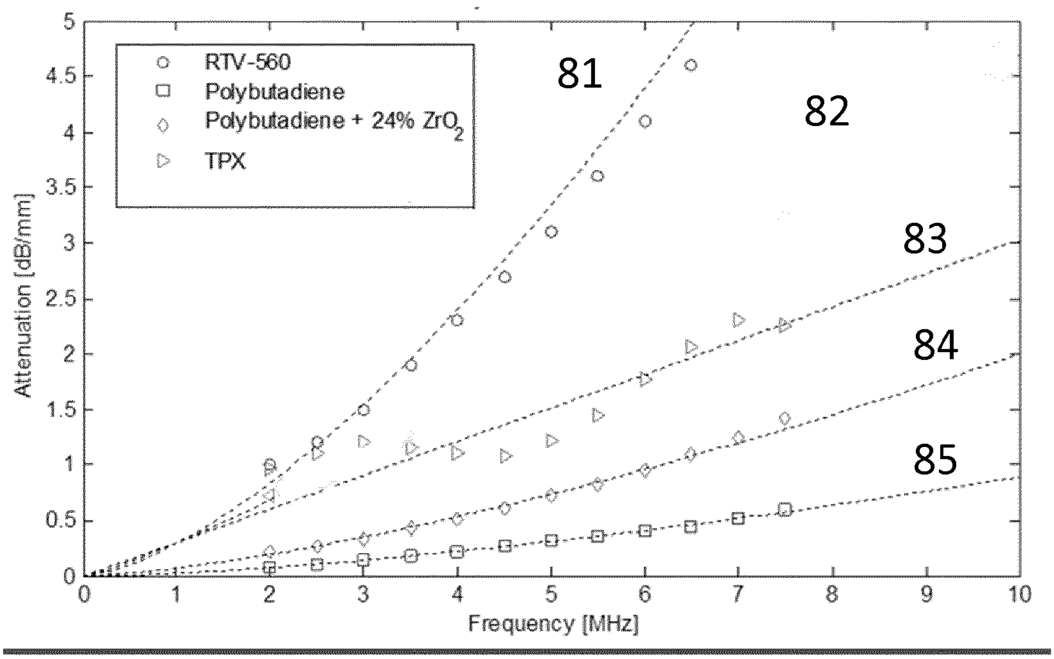

RTV-560
Polybutadiene
Polybutadiene + 24% ZrO2
TPX
Attenuation [dB/mm]
Frequency [MHz]
81
82
83
84
85


FIG. 6

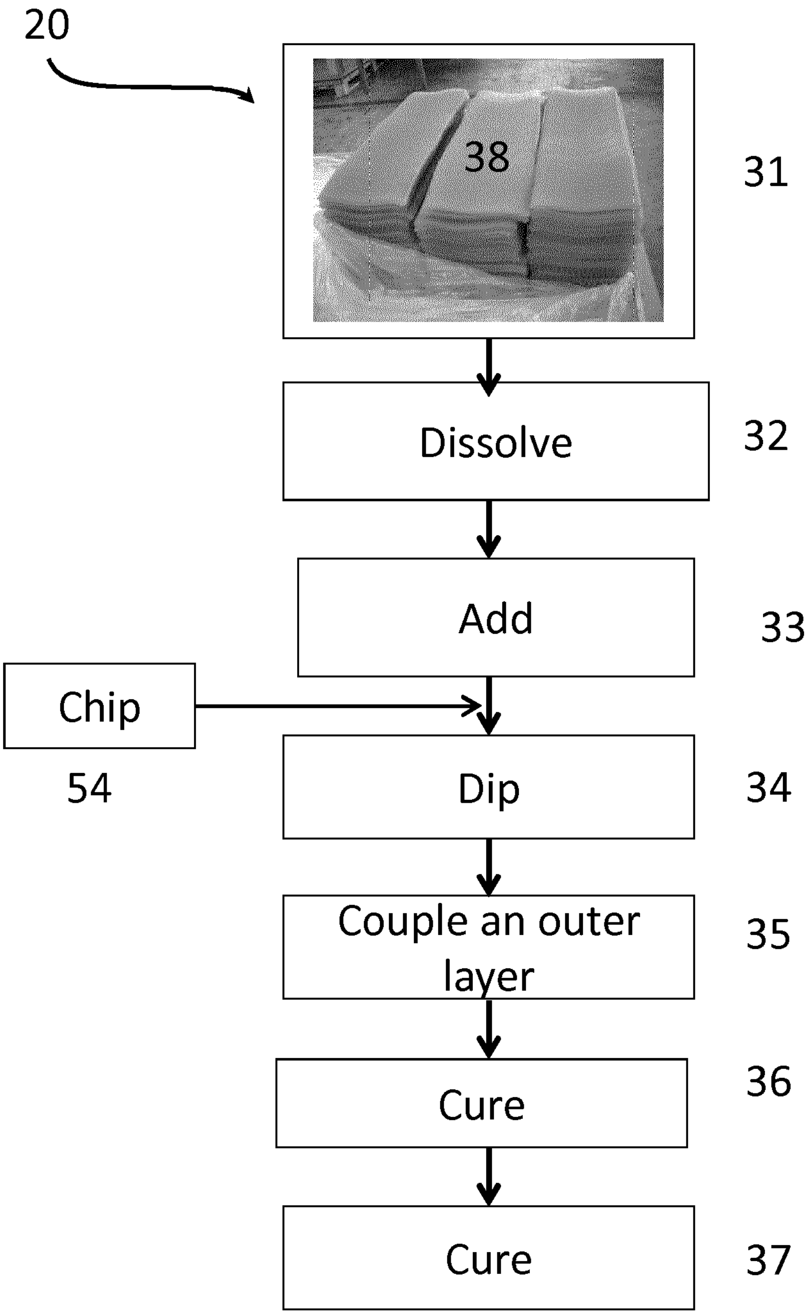
20
38
31
Dissolve
32
Add
33
Chip
54
Dip
34
Couple an outer layer
35
Cure
36
Cure
37

FIG. 7

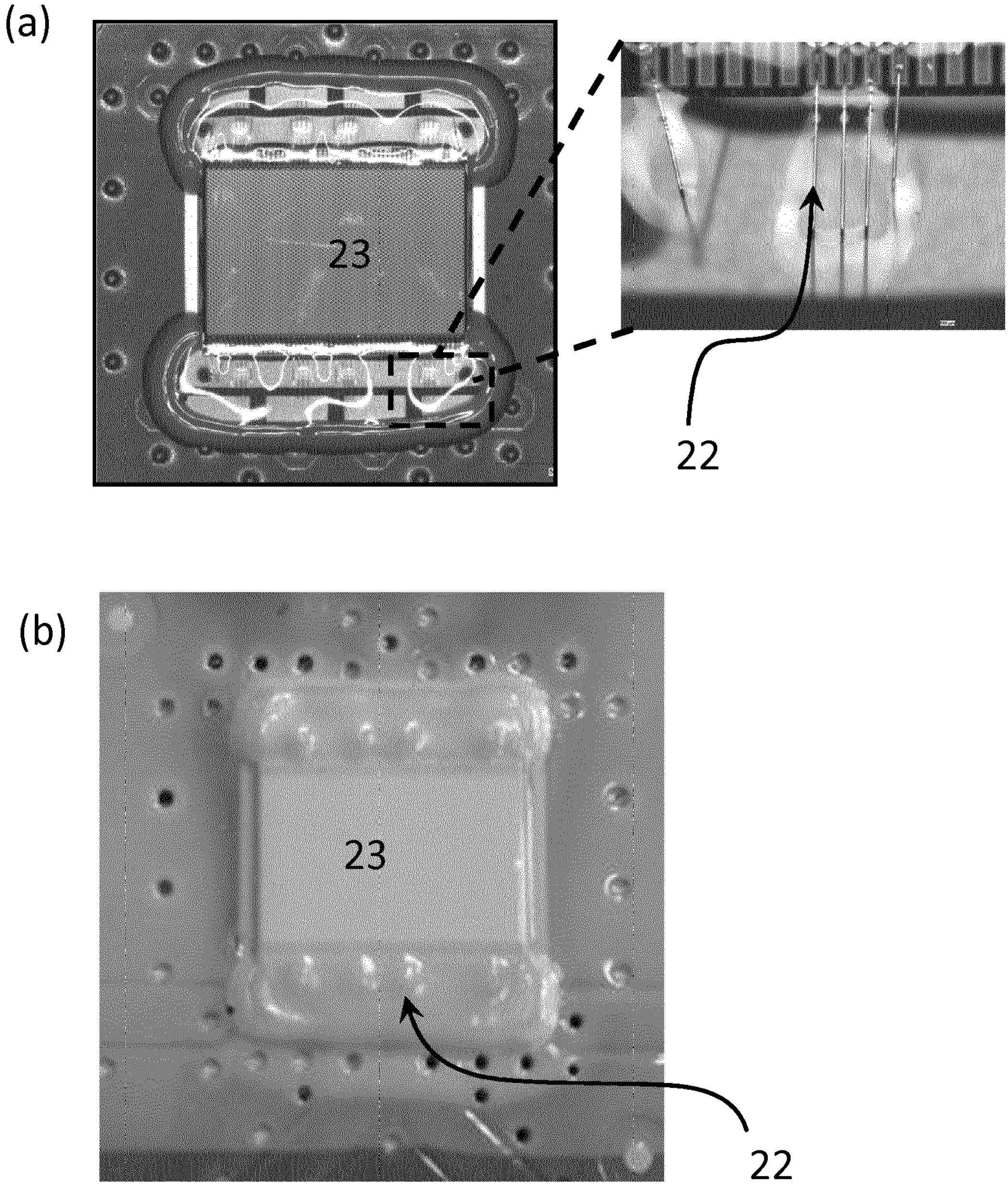


FIG. 8

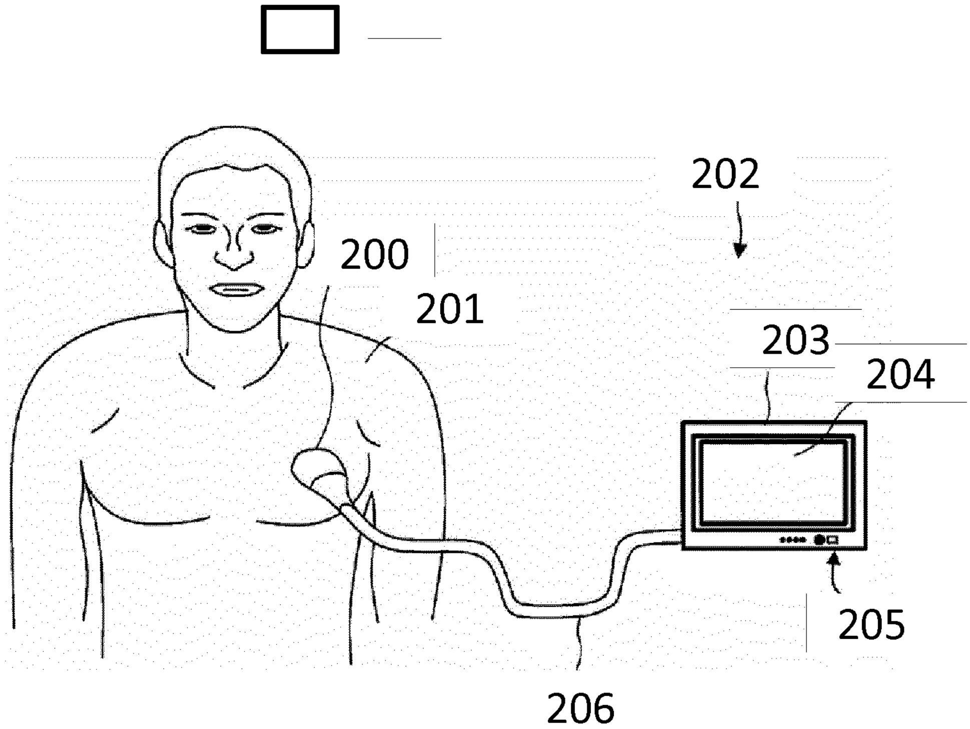


FIG. 9

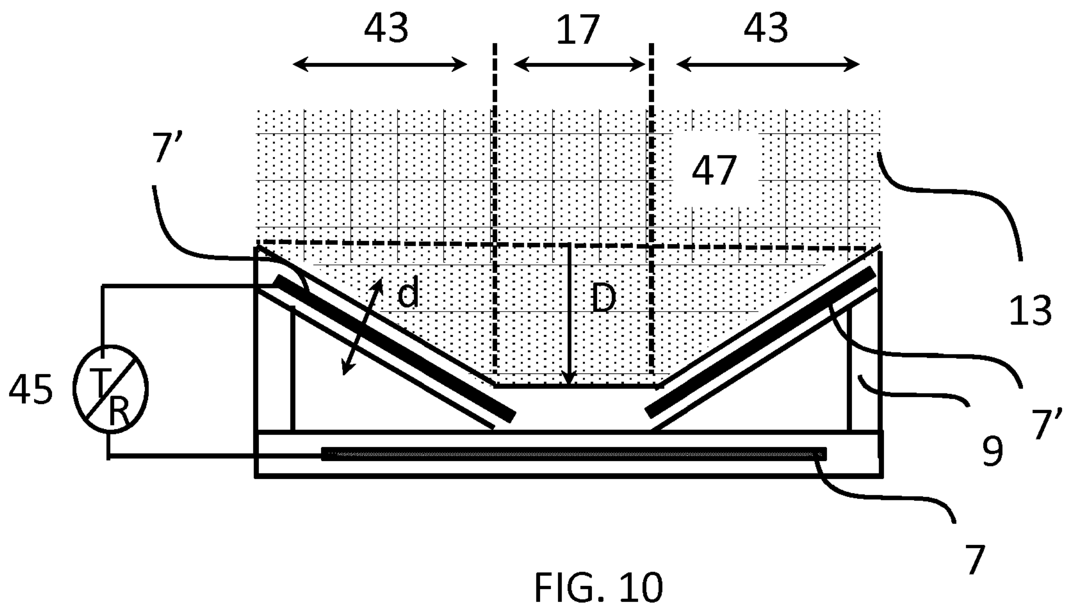


FIG. 10

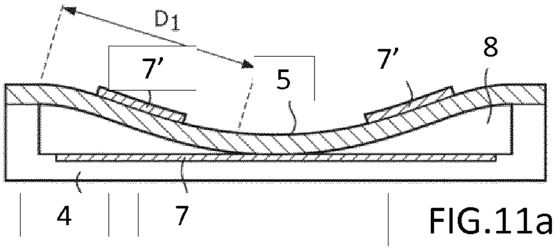


FIG.11a

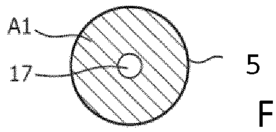


FIG.11b

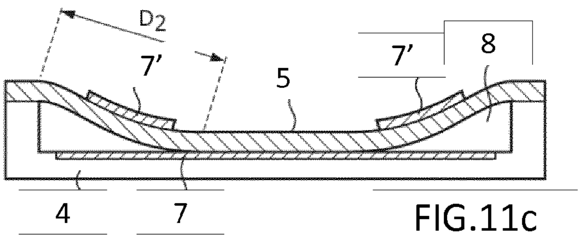


FIG.11c

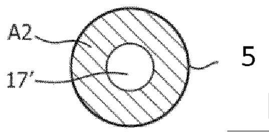


FIG.11d

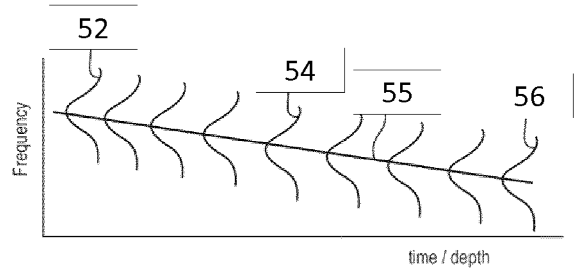


FIG.12

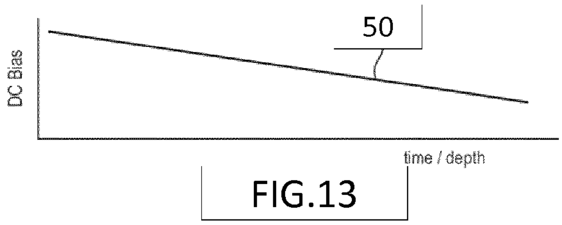


FIG.13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20130301394 A1
- US 2014265728 A1